Europäisches Patentamt

European Patent Office

Office européen des brevets

(19)

(11) Veröffentlichungsnummer: **0 415 874 A1**

# (12) EUROPÄISCHE PATENTANMELDUNG

(21) Anmeldenummer: 90810520.8

(22) Anmeldetag: 09.07.90

(51) Int. Cl.5: **A61F 2/08, A61B 19/00**

(30) Priorität: 28.08.89 CH 3108/89

(43) Veröffentlichungstag der Anmeldung:
06.03.91 Patentblatt 91/10

(84) Benannte Vertragsstaaten:
AT CH DE FR GB IT LI

(71) Anmelder: GEBRÜDER SULZER
AKTIENGESELLSCHAFT
Zürcherstrasse 9
CH-8401 Winterthur(CH)

Anmelder: Protek AG
Stadtbachstrasse 64
CH-3001 Bern(CH)

(72) Erfinder: Jakob, Roland Peter, Prof.Dr.-med.
Aumattweg 35
CH-3032 Hinterkappelen(CH)
Erfinder: Freudiger, Stefan,
Dipl.-Masch.Ing.ETH
Bündackerstrasse 67
CH-3P47 Bremgarten(CH)
Erfinder: Koch, Rudolf
Oberdorfstrasse 229
CH-8257 Berlingen(CH)
Erfinder: Flückiger, Hans
Forchstrasse 190
CH-8128 Hinteregg(CH)

(54) Schlauchartige Durchziehhilfe.

(57) Eine, aus einem aufziehbaren Maschenwerk (1)
bestehende Durchziehhilfe umhüllt mehrsträngige
Prothesen (6) für das Durchziehen durch eine Knochenbohrung (5). Damit wird die Arbeit des Operateurs erheblich erleichtert.

Fig. 3

# SCHLAUCHARTIGE DURCHZIEHHILFE

Die Erfindung betrifft eine schlauchartige Durchziehhilfe für das Durchziehen von Bänder- oder Sehnenprothesen durch einen Knochendurchgang.

Es ist bekannt, als Ersatz für nicht mehr brauchbare Bänder autologe (patienteneigene) "Prothesen" zu benutzen, die dem Patienten erst während der Implantationsoperation entnommen werden. Beispielsweise wird als Kreuzbandersatz häufig ein Stück der Patella-Sehne als Prothese verwendet. Um derartige organische Prothesen nicht zu überlasten, ist man weiterhin dazu übergegangen, parallel zu organischen noch eine künstliche Bänder- oder Sehnen-Prothese, beispielsweise in Form eines geflochtenen oder gewebten textilen Bandes aus Kunststoff, z.B. aus einem Polyester, zu implantieren.

Derartige Bänder-Prothesen verlaufen häufig teilweise in Bohrungen oder Durchgängen durch den angrenzenden Knochen. Es hat sich nun gezeigt, dass das Durchziehen durch den Knochen bei einer zwei- oder mehrsträngigen Prothese, die - wie erwähnt - beispielsweise aus einem organischen und einem künstlichen Strang bestehen kann, für den Operateur häufig mühsam und zeitaufwendig ist.

Aufgabe der Erfindung ist es, die Schwierigkeiten des Operateurs beim Durchziehen mehrsträngiger Prothesen durch einen Knochen zu vermindern.

Erfindungsgemäss wird diese Aufgabe gelöst durch ein aufziehbares, textiles Maschenwerk, das eine mehrsträngige Bänder- oder Sehnenprothese umhüllt, aus mindestens einem durchgehenden Faden besteht und an einem Ende mit einem ausziehbaren Endfaden gesichert ist.

Mit der neuen Durchziehhilfe, die nach Ausziehen des Endfadens durch Ziehen an ihrem durchgehenden Faden "aufgelöst" wird, werden die Stränge der Prothese wieder zu einem Strang vereinigt, der sich leicht durch eine Knochenbohrung ziehen lässt.

Das "Auflösen" der Durchziehhilfe hat sich als besonders einfach erwiesen, wenn das Maschenwerk ein Gestrick ist. Weiterhin lässt sich das Einfädeln der verschiedenen Prothesenstränge in die Durchziehhilfe erleichtern, wenn das Maschenwerk über einen sterilisierbaren, formstabilen Hohlkörper gezogen ist.

Die bevorzugte Anwendung der neuen Durchziehhilfe ist das Durchziehen von einer mindestens aus einem künstlichen Hilfsband und einem organischen, patienteneigenen Band oder einer patienteneigenen Sehne bestehenden mehrsträngigen Prothese.

Im folgenden wird die Erfindung anhand eines Ausführungsbeispiels im Zusammenhang mit der Zeichnung näher erläutert.

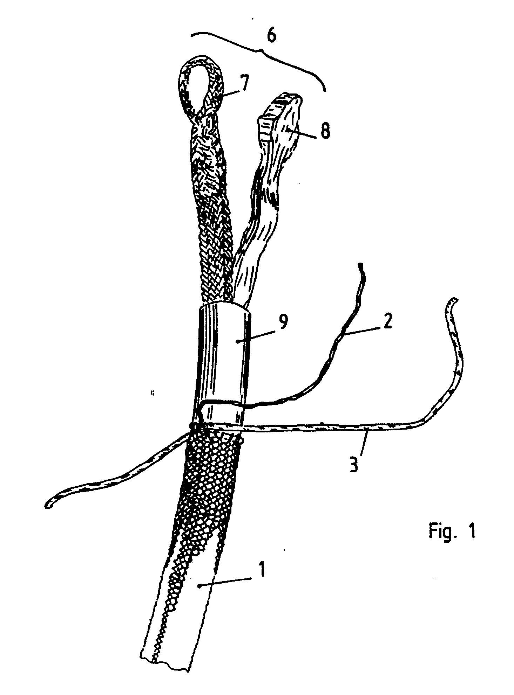

Fig. 1 zeigt die Durchziehhilfe über einen sterilisierbaren, formstabilen Hohlkörper gezogen;

Fig. 2 ist eine Darstellung des zum Durchziehen fertigen Paketes einer erfindungsgemässen Durchziehhilfe mit einem organischen ·und einem künstlichen Prothesenstrang;

Fig. 3 schliesslich gibt schematisch die in eine Knochenbohrung eingezogenen Prothesenstränge wieder, die teilweise noch von der Durchziehhilfe umschlossen sind.

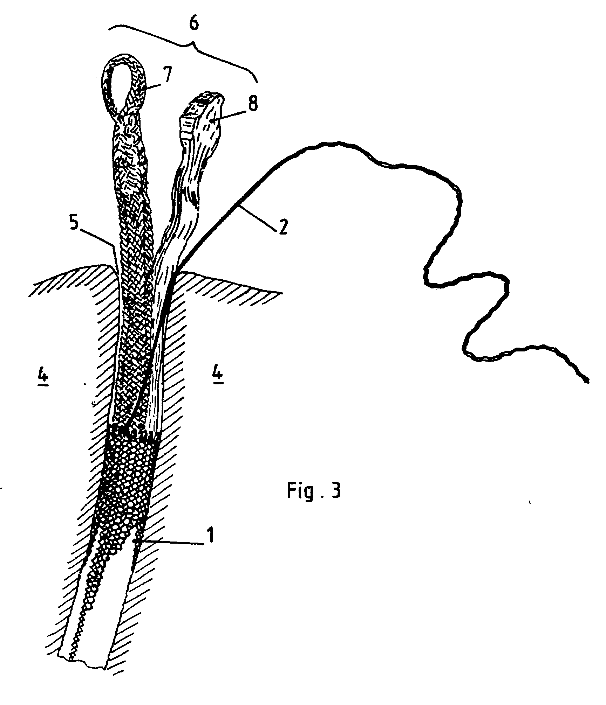

In dem gezeigten Beispiel besteht das Maschenwerk 1 der Durchziehhilfe aus einem einlagigen Gestrick, dessen Maschen aus einem einzigen Faden 2 hergestellt sind. Die Endmaschen des Gestricks sind auf einem Endfaden 3 gehalten. Beide Fäden 2 und 3 weisen relativ lange, freie Enden auf, die nach dem Durchziehen der Durchziehhilfe durch den Knochen 4 (Fig. 3) aus der Knochenbohrung 5 (Fig. 3) herausragen.

Als Materialien für die Fäden können in erster Linie in der Implantat-Technik erprobte und bewährte Kunststoffe, z.B. Polyäthylen, Polyester oder Polyamide, dienen; es ist jedoch auch möglich, die Durchziehhilfe aus chirurgischem Nahtmaterial herzustellen.

Zum intraoperativ erfolgenden Einfädeln einer zweisträngigen Prothese 6, die im vorliegenden Beispiel aus einem textilen, beispielsweise gewobenen oder geflochtenen, künstlichen Hilfsband 7 aus einem Kunststoff, z.B. ebenfalls aus Polyester, und einem autologen organischen Sehnenstück 8 - z.B. einem Stück der Patella-Sehne -besteht, ist das Maschenwerk 1 der Durchziehhilfe über einen formstabilen Hohlkörper 9 gezogen. In diesen Hohlkörper 9 werden die beiden Prothesenstränge 7 und 8 eingezogen, ehe der aus einem sterilisierbaren Werkstoff -z.B. ebenfalls einem geeigneten Kunststoff, wie wiederum Polyester, Polyäthylen oder ein Polyamid - bestehende Hohlkörper entfernt wird.

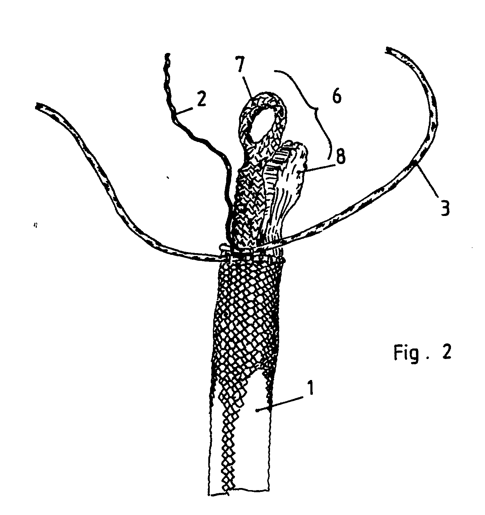

Nach dem Entfernen des Hohlkörpers steht dem Operateur, wie in Fig. 2 gezeigt, ein aus einem Strang bestehender Schlauch zur Verfügung, der relativ einfach durch die Knochenbohrung 5 gezogen werden kann.

Nach dem Durchziehen dieses Schlauches durch die Bohrung 5 zieht der Operateur zunächst den Endfaden 3 aus den Endmaschen des Gestrickes 1; anschliessend wird die Durchziehhilfe durch Ziehen am freien Ende ihres Fadens 2 "aufgelöst" (Fig. 3), so dass durch den Knochen 4 schliesslich nur die beiden Prothesenstränge 7 und 8 verlaufen.

**Ansprüche**

1. Schlauchartige Durchziehhilfe für das Durchziehen von Bänder- oder Sehnenprothesen durch einen Knochendurchgang, gekennzeichnet durch ein aufziehbares, textiles Maschenwerk (1), das eine mehrsträngige Bänder- oder Sehnenprothese (6) umhüllt, aus mindestens einem durchgehenden Faden (2) besteht und an einem Ende mit einem ausziehbaren Endfaden (3) gesichert ist.

2. Durchziehhilfe nach Anspruch 1, dadurch gekennzeichnet, dass das Maschenwerk (1) ein Gestrick ist.

3. Durchziehhilfe nach Anspruch 1 oder 2, dadurch gekennzeichnet, dass das Maschenwerk (1) über einen sterilisierbaren, formstabilen Hohlkörper (9) gezogen ist.

4. Verwendung der Durchziehhilfe nach einem der Ansprüche 1 bis 3 zum Durchziehen einer mindestens aus einem künstlichen Hilfsband (7) und einem organischen, patienteneigenen Band oder einer patienteneigenen Sehne (8) bestehenden, mehrsträngigen Prothese (6).

Fig. 1

Fig . 2

Fig . 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 223 370 (SEEDHORN)(27-05-1987) <br> * Seite 6, Zeile 26 - Seite 7, Zeile 30; Figuren 1,3,5,8 * <br> – – – | 1,4 | A 61 F 2/08 <br> A 61 B 19/00 |
| A | DE-U-8 706 263 (SCHMIEDIG)(26-11-1987) <br> * Seite 4, Zeilen 11-18; Fig. * <br> – – – | 1,4 | |
| A | DE-A-2 607 753 (SPAICHINGEN)(01-09-1977) <br> * Seite 6, Zeilen 4-13 * <br> – – – – – | 1 | |

| RECHERCHIERTE SACHGEBIETE (Int. Cl.5) |
|---|
| A 61 F <br> A 61 B <br> D 04 B |

*Der vorliegende Recherchenbericht wurde für alle Patentansprüche erstellt*

| Recherchenort | Abschlußdatum der Recherche | Prüfer |
|---|---|---|
| Den Haag | 30 November 90 | MOERS R.J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument